# EUROPEAN PATENT APPLICATION

(11) **EP 4 470 592 A1**
(43) Date of publication of application: **04.12.2024**
(21) Application number: 23176706.2
(22) Date of filing: 01.06.2023
(51) Int. Cl.: A61M 25/00, A61M 27/00

(54) **A PACKAGE FOR A MEDICAL DEVICE WITH A CARRAGEENAN BARRIER FILM**

(71) Applicant: Wellspect AB, 431 21 Mölndal (SE)
(72) Inventor: Westman, Gunnar, 438 92 Härryda (SE); Gunnarsson, Maria, 413 23 Göteborg (SE)
(74) Representative: Lind Edlund Kenamets Intellectual Property AB

(57) **Abstract**

A medical device assembly comprises a medical device (1), and preferably a hydrophilic medical device, and a package (3) forming an interior cavity. The cavity is arranged to enclose at least a part of the medical device. The package comprises a substrate made of a bio-based material, such as paper, and a film made of carrageenan arranged on the substrate. The film may further comprise an azetidinium salt bonded to or mixed with the carrageenan, to increase hydrophobicity. Additionally, or alternatively, the film may comprise a plasticizer, such as sorbitol or glycerol.

## Description

### TECHNICAL FIELD OF THE INVENTION

The present invention relates to a medical device assembly comprising a medical device, and preferably a hydrophilic medical device, and more preferably a hydrophilic catheter, such as a hydrophilic urinary catheter, and a package enclosing at least a part of the medical device.

### BACKGROUND OF THE INVENTION

Hydrophilic medical devices are used for many different purposes. For example, hydrophilic urinary catheters are commonly used as intermittent urinary catheters to drain urine from the bladder. Such catheters are wetted and activated prior to use by being immersed in water, or being wetted in other ways. For example, such urinary catheter may be arranged in a wetted, ready-to-use state in the package. Alternatively, the catheters may be packaged together with a wetting fluid compartment, allowing the wetting fluid to be released immediately prior to use.

Catheters are commonly used for draining bodily fluids, e.g. from the bladder. Urinary catheters are e.g. used by a large group of persons for intermittent catheterization, which is a daily-life procedure, taking place several times a day. Typically, catheters for intermittent catheterization are used by patients suffering from urinary incontinence or by disabled individuals like para- or tetraplegics. Using an intermittent catheter, the bladder may be drained through a natural or artificial urinary canal. Many catheters for intermittent catheterization are provided with a hydrophilic coating or the like, providing a smooth and slippery surface when wetted, for safe and comfortable insertion in the urinary canal.

Many hydrophilic catheter assemblies include a supply of wetting fluid, either in direct contact with the catheter or in a separate compartment, for clean and convenient activation of the hydrophilic surface before use.

Such catheter assemblies are e.g. disclosed in US 8459455, US 10384033 and US 8808275. In these catheter assemblies, the activation of the catheter is obtained e.g. by pressing at the bottom of the catheter packaging. The activation aims to wet the catheter with sterile water on the inside of the packaging to swell the hydrophilic coating on the catheter to make it slippery and by that simplify the insertion of the catheter and minimize trauma on the urethra. The packaging is here typically a plastic packaging, or a laminate of plastic and metal film or a metal oxide layer, AlOx, or the like. The packaging that encloses the catheter is also a part of the medical device as it encapsulates the liquid during activation. Another way to release the sterile water and activate the catheter coating is to fold the packaging, thus the packaging also needs to have sufficient mechanical durability. The catheter is used within six minutes and is then disposed of together with its packaging after a single use, which is why a more sustainable alternative to plastic packaging would be of interest.

Today a lot of single use packaging is made in plastic materials from non-renewable resources and are a big contributor to climate change. Since plastic is made from oil, it cannot decompose in nature. In Sweden, a big part of the plastic used is collected and at the end of its cycle it is incinerated to extract the energy. Recycling of plastic is preferred to increase the amount of loops the raw material can be part of before going to incineration. However, recycling of plastic is not performed to any large extent yet and in the end the incineration results in emissions of CO2. Moreover, not all plastic waste is collected and recycled or incinerated but instead ends up in nature which results in microplastics being released, harming the environment. Additionally, these microplastics have a potential toxic effect. Due to these disadvantages, bio-based materials, such as paper, would seemingly be a better alternative than plastic due to its existing infrastructure for collection, recycling and reusing as well as its degradability in nature. Since plastic can be used in a wide variety of areas due to its properties, paper has inherent disadvantages, such as being deteriorated when in contact with water.

Bioplastics are another feasible alternative. The benefits of bioplastics are that they are non-toxic, have a shorter degradation time, reduce fossil fuel consumption, and reduce emission of greenhouse gases and reduces the carbon footprint. Additionally, bioplastics are biodegradable and are a promising approach to substitute petroleum-based plastic.

There is therefore still a need for suitable packaging materials that can be used for medical devices in a cost-efficient and environmentally friendly manner. In particular, there is a need for suitable packaging materials for medical devices with certain needs and requirements, such as the need for exposing the medical device to a wetting fluid prior to use.

### SUMMARY OF THE INVENTION

It is therefore an object to provide a medical device assembly which at least alleviates the above-discussed drawbacks of the prior art. In particular, it is an object to provide a medical device assembly comprising a package which can be produced and recycled in an environmentally friendly manner, and which still efficiently serves the further needs of the medical device assembly, such as enabling a sterile interior, enabling activation of a hydrophilic surface of the medical device when arranged inside the package, etc.

According to a first aspect of the invention there is provided a medical device assembly comprising a medical device, and preferably a hydrophilic medical device, and a package forming an interior cavity, the cavity arranged to enclose at least a part of said medical device, wherein the package comprises an outer substrate made of a bio-based material and a film made of carrageenan arranged on said substrate.

According to another aspect of the invention, there is provided a medical device assembly comprising a hydrophilic urinary catheter and a package forming an interior cavity, the cavity arranged to enclose at least a part of said hydrophilic medical device, wherein the package comprises an outer substrate made of a bio-based material, such as paper, and a hydrophobic film made of linear sulfated polysaccharides, such as carrageenan, wherein the films is arranged facing the interior cavity.

It has been found that carrageenan is well suited to produce a film, which can be made thin and transparent, and with adequate mechanical properties. The film may consequently be used as a protective layer on a substrate material, e.g. made of paper, in order to improve the overall properties of the package. It has also been found that the hydrophobicity of the film can easily be increased by addition of further constituents and modification of the carrageenan, such as addition of azetidinium salt, e.g. to form a water or moisture barrier. It has also been found that the mechanical properties could easily be modified by addition of further constituents, such as a plasticizer or amines, e.g. to increase elasticity, flexibility and/or tensile strength.

It has further been found that a film made of linear sulfated polysaccharides, and in particular carrageenan, is highly efficient in providing a barrier for the cavity formed in the package. The film could be used together with a substrate made of bio-based material, such as paper, which makes the package very environmentally friendly and biocompatible. Further, the film is efficient in providing other advantageous properties, such as enabling and maintaining a sterile environment inside the cavity, allowing wetting of the hydrophilic medical device inside the cavity without destruction or damage to the package, etc. The package can also be produced cost-efficiently.

The film is hereby made as a biobased plastic. Biobased plastic can be made from various types of materials. Generally, proteins, polysaccharides and lipids are used to make bioplastic. Today many bioplastics are derived from biomaterials such as corn, food waste or cereal crops. A crucial aspect of bioplastics is that the material they are produced from is renewable. Advances in research have shown potential for the use of seaweeds as a biomaterial for bioplastic production as they have the ability to form films. There are many advantages of using seaweed as a feedstock due to its high availability and being planted in sea water instead of on land. Additionally, they reduce global warming and ocean acidity due to seaweeds ability to bind CO2 during photosynthesis.

Seaweeds such as algae comprise polysaccharides similar to wood and is therefore a potential source as a bio-based material. Carrageenan is a type of polysaccharide that is found in algae and can be used for bioplastics. Carrageenan is used in many applications for instance it is a common stabilizing agent in food and can used as a film for preserving food. However, pure carrageenan films are hydrophilic and can have weak mechanical properties.

It has now been found that carrageenan can be used as to create a hydrophobic carrageenan barrier film, e.g. for use on the inside of a paper packaging, that can serve as a substitute for the current plastic packaging of urinary catheter assemblies. To obtain a film with improved hydrophobic character and improved mechanical properties, modification with azetidinium salts and addition of plasticizers and amines have been explored. These properties are useable in order to retain the catheter functions.

In intermittent urinary catheters that are wetted immediately prior to use, the exposure for the package to the wetting fluid only occurs within a short time span after activation, and consequently, in such applications, only a short-lived hydrophobic water barrier, e.g. in the time frame of six minutes, is required.

The package may be formed by a first and second sheet material which are connected around the edges, and preferably by means of welding.

The bio-based material, serving as a substrate of the package, is preferably a cellulose based material, and preferably based on paper or seaweed. Such substrate materials, such as paper, are very affordable and cost-efficient, and are also recyclable and environmentally friendly. However, such substrate materials could for many applications not be used as packages on their own, but the addition of the film enables use of such substrates for a great variety of medical assemblies.

It has been found that kappa-carrageenan and iota-carrageenan are particularly suitable for this use, and specifically iota-carrageenan is highly suitable.

The film may further comprise an azetidinium salt bonded to, or mixed with, the carrageenan. It has been found that the properties of film of carrageenan modified with azetidinium salt is greatly improved, e.g. in respect of hydrophobicity. Thus the modified carrageenan film may form a barrier against water and moisture. This is particularly efficient when the film is arranged on the inside of a substrate, such as made by paper.

The azetidinium salt and the carrageenan are preferably arranged to form a cross-linked network.

The azetidinium salt may comprise different R and R' groups. Preferably, the azetidinium salt comprises alkyl groups that are linear or branched and that may contain functional groups such as alcohol, carbonyl groups, nitriles etc. In preferred embodiments, the alkyl groups are branched.

In embodiments, the azetidinium salt comprises at least one of morpholine-azetidinium salt (M-Az), 1,1-dihexyl-3-hydroxy-azetidinium salt (DHA-Az), and 1,1-diallyl-3-hydroxy-azetidinium salt (DAL-Az).

A molar ratio between sulphate in the carrageenan and the azetidinium salt is preferably in the range 1:1 to 2:1.

The film may further comprise a plasticizer. The plasticizer is efficient in providing the desired mechanical properties of the film, such as flexibility, elasticity, tensile strength, etc. By selection of an appropriate plasticizer, and the amount of plasticizer, the mechanical properties of the film could easily be designed to suit various needs and applications. Further, it has been found that in many embodiments the addition of a plasticizer improves the hydrophobic properties of the film.

In a preferred embodiment, the plasticizer is at least one of a polyol, and in particular a sugar alcohol, and a triol, including three hydroxyl groups, and preferably at least one of sorbitol and glycerol, and most preferably glycerol.

However, other plasticizers may also be used. In accordance with an embodiment, the plasticizer is at least one of: glycerol, sorbitol, polyethylene glycol (PEG), and in particular PEG 300, sucrose, fructose, polypropylene glycol (PPG), and water.

The film preferably comprises 5-50 wt% of plasticizer, and preferably 15-50 wt%, and most preferably 20-45 wt%.

In a preferred embodiment, the film is hydrophobic and forms a water barrier. Hereby, the package can be used together with water and other wetting fluids, for activation of the hydrophilic medical device, without any significant degradation or damage to the package.

The film is preferably arranged as an inner film on the substrate, facing the interior cavity. Hereby, the film protects the substrate material from being wetted and potentially damaged by any liquid or fluid inside the interior cavity. However, in alternative embodiments, the film may instead, or additionally, be arranged as an outer film on the substrate, i.e. facing away from the interior cavity.

The hydrophilic medical device may be a hydrophilic catheter, and preferably a hydrophilic urinary catheter. The hydrophilic catheter may e.g. be provided with a coating of a hydrophilic material, such as PVP, but could also be made entirely by a hydrophilic material.

The medical device may further comprise a wetting fluid compartment arranged within said interior cavity. The wetting fluid compartment may e.g. comprise a sachet or pouch containing the wetting fluid. The wetting fluid compartment may be openable by twisting, compression, bending or the like to release the wetting fluid into the interior cavity, thereby to wet and activate the hydrophilic medical device.

However, the package may also be used for accommodation of other types of medical devices, not being provided with hydrophilic coatings or otherwise being hydrophilic. For example, the medical device may in such embodiments be indwelling urinary catheters, stents, rectal catheters, etc.

These and other aspects of the invention will be apparent from and elucidated with reference to the embodiments described hereinafter.

### BRIEF DESCRIPTION OF THE DRAWINGS

For exemplifying purposes, the invention will be described in closer detail in the following with reference to embodiments thereof illustrated in the attached drawings, wherein:
Fig. 1 illustrates an exploded view of a catheter assembly in accordance with an embodiment of the present invention;
Fig. 2 illustrates the assembly in Fig 1 in a use condition;
Fig. 3 illustrates the chemical structures of the dimers for kappa-, iota-, and lambda-carrageenan;
Fig. 4 illustrates helix formation of kappa- and iota-carrageenan forming a gel;
Fig. 5 illustrates the chemical structure of glycerol;
Fig. 6 illustrates the chemical structure of sorbitol;
Fig. 7 illustrates the chemical structure of azetidinium salt;
Fig. 8 illustrates the chemical structures of M-Az, DHA-Az, and DAL-Az, from left to right, respectively;
Fig. 9 illustrates a hypothesized reaction scheme between carrageenan and azetidinium salt;
Fig. 10 illustrates chemical structures of the investigated kappa-and iota-carrageenan with marked functional groups;
Fig. 11 illustrates a FTIR spectra of iota-carrageenan with added azetidinium salt in 1:1 ratio, wherein the dashed line is marked at the reference tops and the spectra is normalized to the peak of maximum absorbance (in the peak range 1024-1035 cm⁻¹);
Fig. 12 illustrates a FTIR spectra of iota-carrageenan with added azetidinium salt in 2:1 ratio, wherein the dashed line is marked at the reference tops and the spectra is normalized to the peak of maximum absorbance (in the peak range 1024-1035 cm⁻¹);
Fig. 13 illustrates a FTIR spectra of kappa-carrageenan with added azetidinium salt in 1:1 ratio, wherein the dashed line is marked at the reference top and the spectra is normalized to the peak of maximum absorbance (in the peak range 1024-1035 cm⁻¹);
Fig. 14 illustrates a FTIR spectra of kappa-carrageenan with added azetidinium salt in 2:1 ratio, wherein the dashed line is marked at the reference top and the spectra is normalized to the peak of maximum absorbance (in the peak range 1024-1035 cm⁻¹);
Fig. 15 is a picture of iota-carrageenan film functionalized with DHA-Az;
Fig. 16 is a picture of a films with added plasticizer, namely iota-carrageenan with 20 wt% added sorbitol;
Fig. 17 illustrates a FTIR spectra of iota-carrageenan with added azetidinium salt and 20 wt% glycerol, wherein the spectra is normalized to the peak of maximum absorbance (in the peak range 1024-1035cm⁻¹);
Fig. 18 illustrates a FTIR spectra of iota-carrageenan with added azetidinium salt and 45 wt% glycerol, wherein the spectra is normalized to the peak of maximum absorbance (in the peak range 1024-1035cm⁻¹);
Fig. 19 illustrates a FTIR spectra of iota-carrageenan with added azetidinium salt and 20 wt% sorbitol, wherein the spectra is normalized to the peak of maximum absorbance (in the peak range 1024-1035cm⁻¹); and
Fig. 20 illustrates a FTIR spectra of iota-carrageenan with added azetidinium salt and 45 wt% sorbitol, wherein the spectra is normalized to the peak of maximum absorbance (in the peak range 1024-1035cm⁻¹).

### DETAILED DESCRIPTION OF CURRENTLY PREFERRED EMBODIMENTS

The package of the present disclosure is particularly useful for hydrophilic urinary catheters, in particular urinary catheters for intermittent use, which are stored in a dry condition and wetted within the package immediately prior to use. Catheters may be used for many different purposes, and for insertion into various types of body-cavities. However, the following discussion is in particular concerned with the preferred field of use, hydrophilic urinary catheters, even though the invention is not limited to this particular type of catheters. Further, the package is also useful for many other medical device assemblies, such as other types of urinary catheter assemblies, assemblies for other types of catheters, as well as for other types of medical devices, and in particular medical devices stored and used in a similar way as hydrophilic urinary catheters. Even though the following description will be mainly focused on hydrophilic urinary catheters, it is to be acknowledged that the disclosure is also relevant for all such other types of medical devices, and also medical devices not being hydrophilic.

It is to be understood that features of the different embodiments are exchangeable between the embodiments and may be combined in different ways, unless anything else is specifically indicated. It may also be noted that, for the sake of clarity, the dimensions of certain components illustrated in the drawings may differ from the corresponding dimensions in real-life implementations of the invention, e.g. the length of the catheter, etc.

The catheter may be an intermittent urinary catheter that is used by female or male users with bladder dysfunction. The catheter may have a hydrophilic coating which is dry before use and therefore needs to be activated with an aqueous fluid, such as water. The activation serves to activate the hydrophilic coating on the catheter which will facilitate the catheterization and prevent trauma on the urethra. Prior to use, the catheter is enclosed by a packaging, which forms a part of the medical device, and e.g. serves to encapsulate the water during activation of the hydrophilic coating and thereby aid the catheterization procedure.

In an embodiment, the catheter assembly comprises a catheter and a package, e.g. a resealable package, accommodating the catheter. Specifically, the catheter may have a hydrophilic surface coating, and the assembly may also include a wetting fluid for activation of the hydrophilic surface coating.

Preferably a wetting fluid is also included in the assembly. The wetting fluid may be arranged in direct contact with the hydrophilic surface of the catheter. However, preferably the wetting fluid is arranged separately from said catheter within the package. The separate arrangement of the wetting fluid can be obtained by means of closed compartment within the package. However, in a preferred embodiment, the wetting fluid is arranged in a wetting fluid container arranged within said package, such as in a pouch, sachet or the like. In case the wetting fluid is arranged separately, the container or compartment may be openable into the part of the package housing the catheter, in order to enable release of the wetting fluid into contact with the hydrophilic part of the catheter before use. Release of the wetting fluid can be obtained by squeezing, bending or the like, as is per se well known in the art.

In a preferred embodiment, the package is elongate, and preferably with an essentially rectangular form. Hereby, a very compact product is obtained.

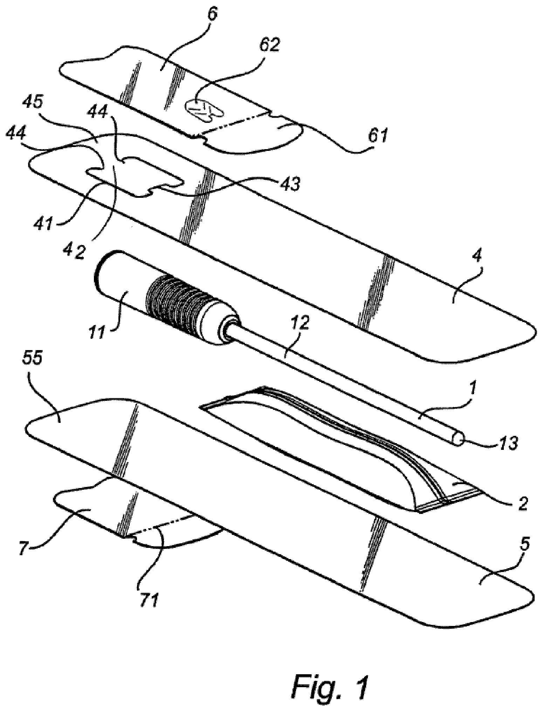

The catheter assembly as illustrated in Figs. 1-2 comprises a catheter 1 having a hydrophilic surface coating, a wetting fluid for activation of said hydrophilic surface coating and a package 3 accommodating the catheter and the wetting fluid.

The catheter 1 may be any type of hydrophilic catheter, as is per se well known in the art. Preferably, the catheter may comprise a flared rearward portion, forming a flared connector 11, and an elongated shaft 12, connected to the flared connector 11, and in the opposite end having a catheter insertion end 13.

At least a part of the elongate shaft 12 forms an insertable length to be inserted through a body opening of the user, such as the urethra in case of a urinary catheter. By insertable length is normally, in the context of a hydrophilic catheter, meant that length of the elongate shaft 12 which is coated with a hydrophilic material, for example PVP, and which is insertable into the urethra of the patient. Typically, this will be 50-140 mm for a female patient and 200-350 mm for a male patient. Even though PVP is the preferred hydrophilic material, other hydrophilic materials may be used, such as hydrophilic polymers selected from polyvinyl compounds, polysaccharides, polyurethanes, polyacrylates or copolymers of vinyl compounds and acrylates or anhydrides, especially polyethyleneoxide, polyvinyl-pyrrolidone, heparin, dextran, xanthan gum, polyvinyl alcohol, hydroxy propyl cellulose, methyl cellulose, copolymer of vinylpyrrolidone and hydroxy ethylmethyl acrylate or copolymer of polymethylvinyl ether and maleinic acid anyhydride. The coating may also comprise an osmolality-increasing compound, as is e.g. taught in EP 0 217 771

The wetting fluid is preferably arranged separated from the catheter, in a wetting fluid container 2, such as a pouch or a sachet. The wetting fluid container is openable by means of e.g. exerting a pressure to the container, whereby the wetting fluid is released into the package, thereby wetting the hydrophilic surface of the catheter. The wetting fluid is preferably an aqueous liquid, such as water or saline. Such wetting fluid containers and wetting fluids are per se well known in the art. The wetting fluid container can e.g. be made of sheet material comprising aluminium.

The wetting fluid may be any fluid that wets a hydrophilic surface of the catheter, such as pure water, saline, etc.

Preferably, the wetting fluid container 2 is arranged close to the insertion end of the catheter, whereby a compact catheter assembly can be obtained.

The package may be formed in various ways, such as in the form of a tube, etc. In a preferred embodiment, the package comprises a first sheet material 4 and a second sheet material 5, connected around the edges to form an inner cavity housing the catheter and the wetting fluid. The first and second sheet materials are preferably connected around the edges by means of welding. Preferably, the first and second sheet materials comprise sheets having weldable inner layers.

In an embodiment, the package comprises a resealable opening. In such an embodiment, the first sheet material may comprise a perforation line 41 extending along a non-closed loop defining a flap opening. The non-closed loop, defining a flap opening, is preferably arranged over the connector end of the catheter 1. Further, the non-closed loop preferably has an opening 42 debouching towards the end of the package being opposite to the insertion end of the catheter. At the other end, the non-closed loop preferably forms a tongue 43 directed inwardly towards the non-closed opening of the non-closed loop. The loop ends, at the opening 42, are preferably directed towards the interior of the non-closed loop. The non-closed loop may generally form a C- or U-shape.

Over the non-closed loop, a third sheet material 6 may be arranged, and connected to the first sheet material 4 by means of an adhesive. The third sheet material is here arranged to cover the entire flap opening with a margin, preferably exceeding 2 mm, and most preferably exceeding 5 mm. An end of the third sheet material is not adhered to the first sheet material, and forms a tab 61 providing a grip portion for peel opening of the package. The tab is preferably arranged in the end directed towards the insertable part of the catheter.

The third sheet material further preferably comprises a weakened area 62 forming a seal integrity mark. Hereby, it is ensured that the seal has not been broken before use, ensuring full integrity of the product. Preferably, the seal integrity mark is arranged between the tab and the part of the third sheet material overlying the perforation line. The weakened area preferably comprises weakened or perforated lines arranged in a pattern, e.g. as illustrated in the drawings, making part of the third sheet material to remain adhered to the first sheet material during peel off of the third sheet material.

The catheter assembly may further comprise a fourth sheet material 7 arranged on the second sheet material, i.e. on the side of the package being opposed to the third sheet material. The fourth sheet material is also connected by means of an adhesive to the package, and forms a tab 71 not provided with adhesive, said tab providing a grip portion for exposure of said adhesive to form a holding arrangement for the package. By means of this fourth sheet material, the catheter assembly may e.g. be attached to a sink, a wall or the like, which enables very efficient and easy handling of the product, even for users with reduced dexterity.

In a preferred embodiment, the package is elongate, and preferably with an essentially rectangular form. Hereby, a very compact medical device assembly is obtained. It is further preferred that the short side 45, 55 of the elongate package, being closest to the flap opening, has an inwardly protruding shape. Hereby wrinkling, buckling and the like of the package is avoided, ensuring a tight seal by means of the third sheet material.

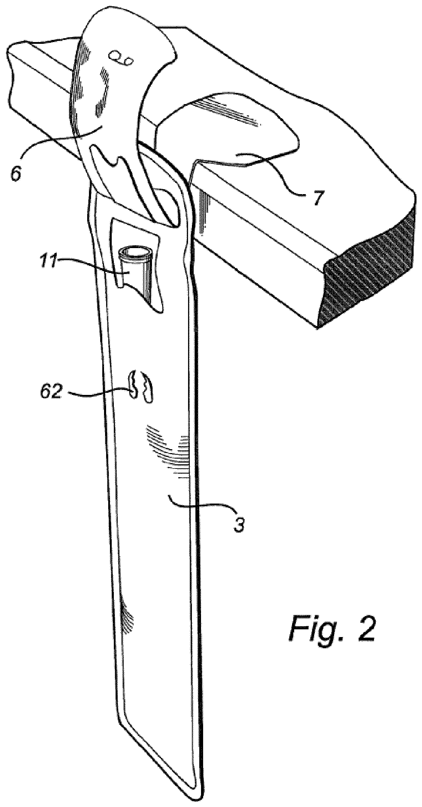

In use, the wetting fluid container is opened, for activation of the hydrophilic surface of the catheter. After sufficient wetting, the tab of the fourth sheet may be peeled, so that the catheter assembly can be connected to a sink or the like (see Fig. 2). The tab of the third sheet is peeled open, and the catheter is removed and used. Thereafter, the catheter may be reinserted, and the package can then be closed, and stored for later disposal.

However, the catheter need not be reinserted into the package, and may be discarded immediately, together with the package. In such embodiments, the package need not to be reclosable. In such embodiments, other opening means may also be provided, such as peel openings, tear openings and the like.

The package of the medical device assembly will be discussed in more detail in the following. The medical device assembly discussed in the foregoing, with reference to Figs. 1 and 2, generally corresponds to the package disclosed in US 8459455, said document hereby incorporated in its entirety by reference. However, the package of this disclosure may also be used in other types of medical device assemblies, such as the packages disclosed in US 10384033 and US 8808275, said document hereby also being incorporated in their entirety by reference. In all these assemblies, the medical device is arranged within the package together with a supply of wetting liquid arranged in a separate wetting fluid compartment, such as a pouch or sachet. The package material of the present disclosure has been found to be of particular great advantage in respect of such medical device assemblies, and the film could here serve as a protection for the substrate of the package, and e.g. be arranged on the inside of the catheter package, to hinder water to wet the paper or other material used as the substrate.

However, other types of assemblies can also be used, such as assemblies not including any supply of wetting fluid, and assemblies where a wetting liquid is arranged directly together with the medical device, without any separate wetting fluid compartment.

To make the product environmentally friendly, it is of advantage to use a package material which is not made of plastic and other crude oil-based materials. From an environmental point of view, it is desirable to find a more sustainable packaging material for an intermittent catheter while being able to preserve its full function.

It has been realized that bio-based materials such as paper and seaweeds are both promising and potential candidates as sustainable packaging materials. Both paper and seaweeds materials are built up by polysaccharides which makes them compatible with each other as packaging material. One of the more interesting polysaccharides derived from seaweed is carrageenan. It has now been found that carrageenan may be used to create a hydrophobic film for the potential use on the inside of a paper packaging that will substitute the plastic packaging in the catheter assembly. Two types of carrageenan, kappa- and iota-carrageenan, have been evaluated for this purpose. To increase the hydrophobicity of the films, modification with azetidinium salt has been use. Furthermore, to improve the films mechanical properties addition of plasticizers has been made.

Experimental results show that modification with azetidinium salts improved the hydrophobicity of iota-carrageenan films which indicates that a reaction has occurred. There was no indication of reaction between kappa-carrageenan and azetidinium salt as no increase in hydrophobicity could be observed. The addition of plasticizers fulfilled its intended purpose on the pure kappa- and iota-carrageenan films and at the same time increased the hydrophobicity of the films.

The addition of plasticizers to the iota-carrageenan films modified with azetidinium salt could in some occasions infer slightly with the hydrophobic effect observed for the modified film without plasticizer, but on the other hand significantly improved the mechanical properties. In other occasions, the addition of a plasticizer improves the hydrophobicity.

In the following, focus is primarily directed to two types of carrageenan, viz. iota-carraggeenan and kappa-carrageenan, which have been found to be particularly suitable for forming a film, whereas lambda-carrageenan is excluded from the following discussion, since it is more difficult to form into a film.

### Properties and Characteristics of Carrageenan Polysaccharide derived from seaweeds

Seaweeds are a non-flowering organism called macroalgae and are found in both seas and oceans all over the globe which are occupying 71% of the globes total area. The availability of this feedstock is thus abundant. Seaweeds are known for their high content of minerals, vitamins, polysaccharides and bioactive substances like lipids and proteins. The large content of polysaccharides is produced in the growing process of the seaweed and can be extracted for further use.

The structure of polysaccharides is defined by long chains of simple sugars called monomers, which are linked together with glycosidic bonds. Polysaccharides can vary in many ways in terms of altering monomers and chain length. The molecular structure of polysaccharides can be linear or highly branched. Additionally, polysaccharides can be composed of either heteropolysaccharides or homopolysaccharides, further adding to the diversity of their molecular composition. Polysaccharides are eco-friendly and versatile. The polysaccharides may alter in conformation and configuration, depending on the composition, and in particular the sequences of type of monomers.

Carrageenans are a family of natural linear sulfated polysaccharides that are extracted from red edible seaweeds. Carrageenans are widely used in the food industry, for their gelling, thickening, and stabilizing properties. Their main application is in dairy and meat products, due to their strong binding to food proteins.

Carrageenans contain 15-40% ester-sulfate content, which makes them anionic polysaccharides. They can be mainly categorized into three different classes based on their sulfate content. Kappa-carrageenan has one sulfate group per disaccharide, iota-carrageenan has two, and lambda-carrageenan has three. These are the three main commercial classes of carrageenan, but other classes of carrageenan are also in existence and can also be used.

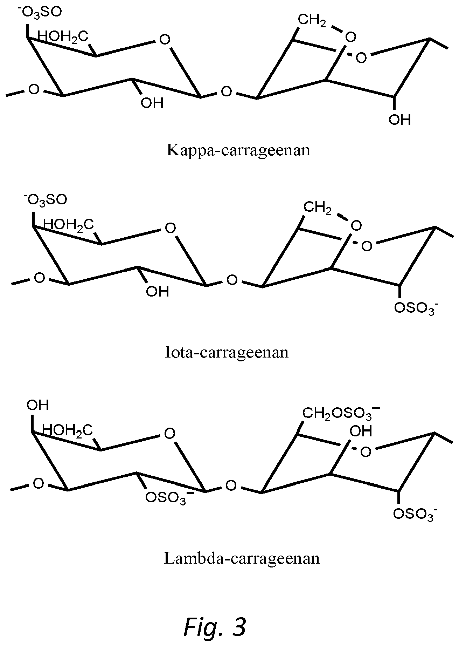

Carrageenan is a collective name for a polysaccharide which is extracted from various types of red algae. This polysaccharide is composed of linear chains, featuring alternating units of 3,6-anhydro-D-galactose and D-galactose, which are sulfonated, rendering it highly hydrophilic. The polysaccharides can comprise different monomer units with different sulphate content and positions. Carrageenan has therefore been classified into three different main groups: Kappa, iota and lambda. The chemical structures of the dimers for kappa-, iota-, and lambda-carrageenan are shown in Fig. 3. The range from one to three sulphate ester units in the dimer of carrageenan provides different properties.

Carrageenan's are known to have useful rheological properties such as their viscoelasticity and viscosity. Additionally, the reactivity of carrageenan is primarily determined by the sulphate groups of half-ester type present within its structure.

The chemical structure for all types of carrageenan provides properties such as water solubility, pH stability, and the ability of the polymer to form high-viscosity solutions. The viscosity increase can be caused by two mechanisms: 1) a decrease in free volume by interaction by linear chains or 2) crosslinking between chains resulting in the formation of a physical gel. Solutions of carrageenan can form a gel, with apparent increase in viscosity. The viscosity of carrageenan depends on the temperature, concentration, and the solvent used. The viscosity increases almost exponentially with increasing concentration, while it decreases with increasing temperature. Since carrageenan is a polysaccharide, its viscosity will also depend on its molecular weight, as this can contribute to an increased entanglement of chains.

The gel formation of carrageenan is an important property in relation to the present disclosure, since it allows carrageenan to evaporate to a film.

A gel is here defined as a crosslinked network structure with weak or strong bonds.

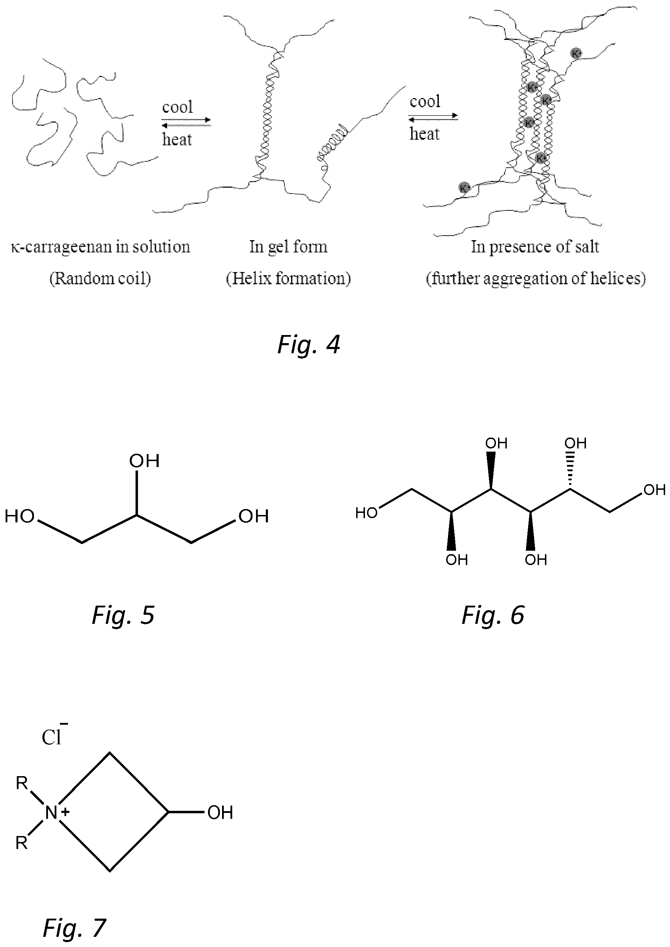

The gel formation can be achieved through a heating and cooling process that allows carrageenan to change structure. The solution forms gel due to a helix formation due to hydrogen bonds between the galactosyl units of the carrageenan structure, which further creates junction zones. The junction zones play an important role as crosslinkers within the network and significantly contribute to the overall structure of the gel. Hot carrageenan solution is in a conformation of random coils, and when the solution cools, it transforms to helical rods in a rigid structure. This process is illustrated in Fig. 4. However, it is primarily kappa- and iota-carrageenan that go through this process while lambda-carrageenan is less prone to undergo gelation. This is because lambda-carrageenan does not have the 3,6-anhydrogalactose unit in the structure, contrary to kappa- and iota-carrageenan. The 3,6-anhydrogalactose unit promotes the rotation in the structure while galactose unit form hydrogen bonds between the chains, resulting in the formation of helices which is beneficial for the gel formation.

For this reason, kappa- and iota-carrageenan are preferred over lambda-carrageenan in relation to the present disclosure, and the use of carrageenan as a protective film in a package of a medical device assembly.

In the carrageenan structure, the strong anionic half-ester is primarily responsible for its reactivity. Since this free acid is present, carrageenan may be provided with a positive counterion such as sodium or potassium. The water solubility depends on the number of sulphate groups as well as the bound cations to these groups.

Seaweed polysaccharides, such as carrageenan, have been found to have very advantageous film forming properties. In addition to the gelling and thickening properties, which provides structure to the film, the film also has good barrier and protective properties. Carrageenan also has certain antibacterial, antioxidant and antiviral properties.

### Carrageenan Extraction and availability

Carrageenan is extracted from the cell wall of red algae. The extraction can be made either by hot water, to obtain a high yield, or alkali treatment for a better gel-formation ability. The general extraction process of carrageenan is to treat a collection of dried red algae in powder-form by either of the two methods, with the general purpose to let the cell walls of the algae swell and get raptured and in this way extract carrageenan. The advantage of the alkali compared to hot water extraction is that it increases the 3,6-anhydro-α-D-galactopyranose content and thereby gives a lower sulphate content, since it modifies the carrageenan precursors into kappa- and iota-carrageenan. Thus, the gel formation is enhanced by the alkali treatment given that kappa- and iota-carrageenan have good properties for gel forming. Subsequently after the extraction process, the diluted carrageenan is filtered to concentrate and remove algal residues.

### Film formation of Carrageenan

Carrageenan can form a film due to its gelling properties. The film is formed from the gel as the solution evaporates, thus the formation of the film is based on the same phenomena of physical entanglements. The film forming process involves different driving forces. One driving force is the electrostatic interaction. Carrageenan contains a negatively charged sulphate ion and by introducing a positive ion, the charge of sulphate is neutralized and the film formation is initiated. Another driving force is the hydrogen bonds present. Carrageenan contains several hydroxyl groups that can form intra- and intermolecular bonds with each other, which is of importance for the film formation due to the double helices that are formed.

Carrageenan films can be shaped by several different methods. For example, a film may be formed by dip coating, which involves immersing a substrate surface in a carrageenan coating solution and then allowing it to air dry, and results in the formation of a film directly on the surface. Dip coating is rather simple to perform, however, it can be difficult to control the thickness of the film.

Another coating method is solvent casting which involves spreading the carrageenan film solution on a mold and letting it dry while the solvent evaporates. It is of advantage to use a mold made of a material that interact as little as possible with the film forming chemicals and polymers. This method is also quite simple to perform and has a low cost, however, since it requires a long drying time, it might not be the best technique to use for large scale production.

### Mechanical properties of carrageenan films and use of plasticizers

Film thickness can be affected by several different conditions. It has been noted that a higher carrageenan concentration will increase the thickness of the film. A higher carrageenan concentration prevents mobility of the molecules in the film forming solution, resulting in the molecules being locked into these positions as the gel dries to form the film. The thickness is also affected by the drying environment. A higher temperature and a longer drying time will result in a thinner carrageenan film. This is because a higher temperature will create a more compact cross section in carrageenan as well as increasing the degree of crystallization.

The mechanical properties of the carrageenan film may be determined by tensile strength and elongation at break. By using the tensile test, a stress-strain curve can be obtained and analyzed to provide the average values of stress and strain. The tensile strength has been found to increase with thickness, while elongation at break and water vapor transport rate exhibit a decrease as the film thickness increases.

Kappa-carrageenan has better mechanical properties in comparison to iota-carrageenan. This is due to its low sulphate content and negative charge. However, the kappa-carrageenan film exhibits a more brittle behavior. Further, a higher concentration of carrageenan will result in a stronger intermolecular interaction which will lead to a loss of elasticity of the film.

To improve the mechanical properties of the carrageenan film, plasticizers can be added. A plasticizer is a low-volatile substance added to a material to improve the flexibility and elasticity. The addition of plasticizers has also been shown to increase film thickness. Further, addition of plasticizers in film formation results in fewer defects and thus improvement of the mechanical properties.

The plasticizer is preferably a triol, such as glycerol, or a sugar alcohol, such as sorbitol.

Two suitable plasticizers for carrageenan have been found to be glycerol and sorbitol. The addition of glycerol and sorbitol to carrageenan speeds up the formation of double helices in the carrageenan structure due to carrageenan being less soluble in glycerol and sorbitol. These helices are more randomly ordered compared to when carrageenan is dissolved in water.

Glycerol may be used as a plasticizer due to it being compatible and stable with biopolymers.

Glycerol may also increase the hydrophobicity of the film. This may be due to glycerol's ability to form stronger hydrogen bonds with polysaccharides than water, which prevents the water molecules to combine with the glycerol and polysaccharides. As mentioned previously, a higher concentration of plasticizer has demonstrated an increase in film thickness. Despite glycerol having a low evaporation rate, it still evaporates at room temperature during film formation, resulting in a loss of glycerol as the film solidifies. Carrageenan does not evaporate at all, thus there is no loss of carrageenan as the films solidifies. Fig. 5 illustrates the chemical structure of glycerol.

An alternative plasticizer is sorbitol, a polyol and sugar alcohol, which is a compound made up of many hydroxyl groups. The chemical structure of sorbitol is shown in Fig. 6. Due to its ability to form hydrogen bonds with polymers containing numerous active OH- and NH-groups, sorbitol is a suitable choice as a plasticizer, effectively enhancing the properties of such polymers. The addition of sorbitol as a plasticizer reduces internal hydrogen bonding in carrageenan, due to sorbitol being able to access between polymer chains, which results in an increased flexibility. The film thickness increases with an increasing sorbitol concentration because sorbitol can access into the carrageenan network which leads to pores in the matrix shrinking. This will in turn result in an obstacle for water to permeate the surface and the film therefore becomes more water resistant.

It has been found that a concentration of plasticizer within 20-45% (w/w) is efficient to form flexible and elastic films made out of bio-materials.

### Modification with Azetidinium salt to obtain a hydrophobic film

Azetidinium salts may be used to modify the functionality or mechanical properties of various organic compounds. In Fig. 7 the general structure of an azetidinium salt is illustrated.

Azetidinium salts are synthesized by reacting either dialkylamines or asymmetric secondary amines with epichlorohydrin, depending on whether a symmetric or asymmetric salt is desired. The salts can either form a ring-closed or ring-open product, depending on the solvent used in synthesis. Using isopropanol as a solvent has shown to result in ring-open structures, while using water as a solvent has shown to result in ring-closed structures.

Azetidinium salts comprise, or consist of, a four membered carbon ring, with a hydroxyl group attached at the third position and a positively charged nitrogen atom at the 1,1-position. There are also two end groups, R' and R", attached to the ring which can be varied depending on desired properties. Azetidinium salts can be divided into two groups, symmetrical and asymmetrical azetidinium salts depending on the structure of their end groups R' and R". The symmetric azetidinium salts comprise two identical end groups, while the end groups of the asymmetrical azetidinium salts may differ in length and or ratio. By varying the end groups of the secondary amines when synthesizing the salt, different properties and steric structures can be obtained.

A wide range of azetidinium salts can be obtained by simply changing the R-groups in the starting material, each possessing distinct characteristics. In relation to the present disclosure three different azetidinium salts were chosen, namely morpholine-, 1,1-dihexyl-3-hydroxy-, and 1,1-diallyl-3-hydroxy-azetidinium salt. The three salts will further be referred to as M-Az, DHA-Az, and DAL-Az, respectively. These three salts were chosen based on their properties and because their structures differ from each other.

M-Az is a ring-closed, symmetrical azetidinium salt which comprises a morpholine functional group attached to the nitrogen at the 1,1-position. Morpholine salt reacts well with fatty acids and increases water resistance.

DHA-Az was chosen due to its long carbon chains, where longer carbon chains increase hydrophobicity. DHA-Az comprises two carbon chains with six carbons in each chain. However, DHA-Az exhibits a lower water solubility than the other two salts.

DAL-Az comprises a secondary amine and two alkene groups. This salt is soluble in water at room temperature and is useable as a solvent in organic synthesis. DAL-Az contains double bonds in its carbon chains which should make it more reactive than for example DHA-Az, which only has single bonds.

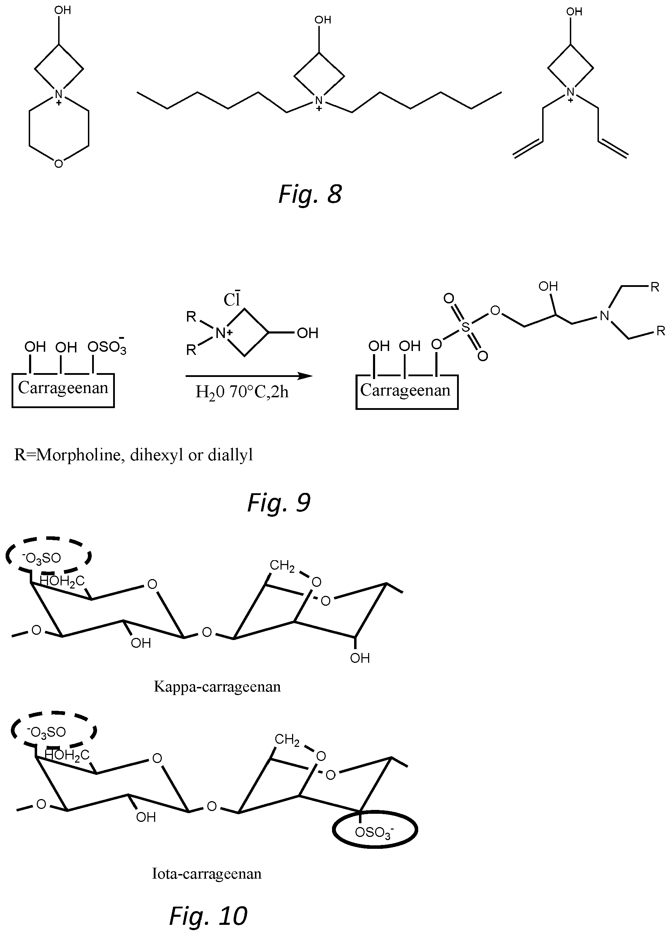

The structures of the three preferred azetidinium salts are shown in Fig 8.

Azetidinium salts react with nucleophiles through the nucleophiles large orbitals which enables the reaction. Sulphate ester groups function as a nucleophile as it possesses the similar arrangement of orbitals and therefore is suitable for a ring opening reaction with azetidinium salts. Sulphate groups can be used as a nucleophile in the ring opening reaction. Since carrageenan naturally contains these sulphate ester groups, azetidinium salt react in the same manner as in e.g. sulfonated nanocrystalline cellulose.

The hypothesized reaction between carrageenan and azetidinium salt is illustrated in Fig. 9.

### Characterization of biobased barrier films

Fourier Transform Infrared Spectroscopy, FTIR, is a characterization method mainly used to analyze and identify different organic substances. FTIR measures absorbance of infrared radiation applied to a sample. FTIR is a versatile technique that can analyze gaseous, liquid, and solid samples and provides real-time measurements. Another advantage with FTIR is that it is a non-destructive characterization method meaning that the samples are not destroyed during analysis. This means that one sample can be analyzed several times. From the FTIR measurements a FTIR spectra is obtained with absorbance peaks and wavenumbers.

The absorbance bands of the peaks in the spectrum can be divided into two groups: group frequencies and molecular fingerprint frequencies.

The group frequencies show the different bonds of typical functional groups in a molecule. These are typically above 1500 cm⁻¹ in the spectra. The molecular fingerprint frequency group shows the frequency of the more specific bonds, and these are typically below 1500 cm⁻¹ in the spectra. However, this region is less reliable since the functional groups can absorb light in this region as well. When interpreting the FTIR- spectrum one usually starts by identifying the functional groups present at the high frequencies. Then the functional groups are typically confirmed by analyzing and confirming the structure of the compound in the sample. There are several FTIR spectra available to use as a comparison when trying to decide which compounds and functional groups are present in a sample.

There are several peaks present in a spectrum of carrageenan. These peaks are presented in Table 1. The above-discussed reaction implies that the reaction involves the sulphate groups in the carrageenan structure. Therefore, the frequencies of the sulphate groups are interesting to study, and these are highlighted in Table 1.

**Table 1: Wavenumbers for different functional groups in carrageenan. Notably, C4-O-S in galactose is present in both kappa-and iota-carrageenan, whereas C2-O-S in 3,6-anhydrogalactose is only present in iota-carrageenan.**

| **Wavenumber (cm⁻¹)** | **Functional group** |
|---|---|
| 3600-3000 | O-H (stretching) |
| 3000-2800 | C-H (stretching) |
| 1645-1640 | Water |
| 1126 | Glycosidic bonds |
| 850-840 | C4-O-S in galactose (stretching) |
| 805-800 | C2-O-S in 3,6-anhydrogalactose |

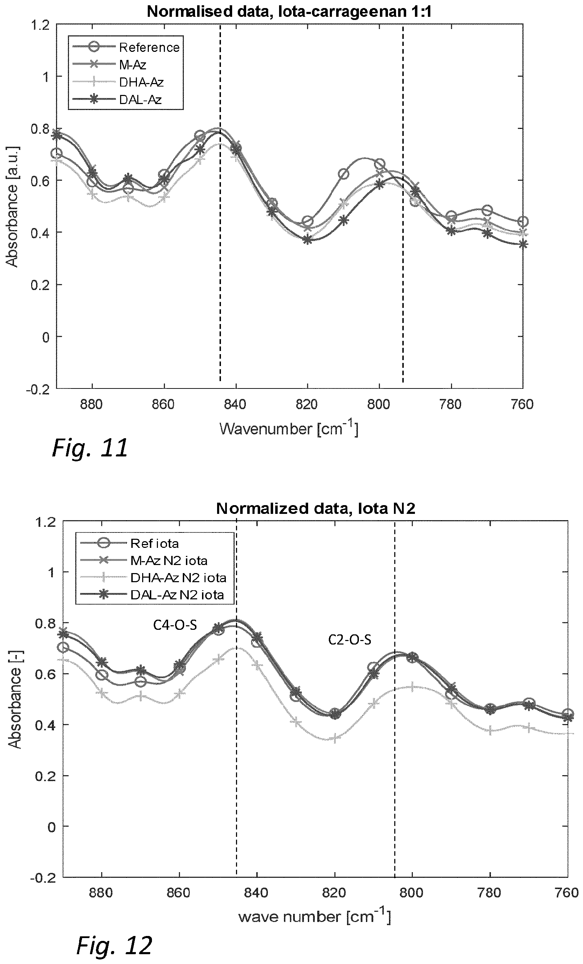

Fig. 10 illustrates the chemical structures of the investigated kappa-and iota-carrageenan with marked functional groups. The sulphate group C4-O-S in galactose is present in both kappa- and iota-carrageenan, circled in dashed line in Fig. 10. However, iota-carrageenan has one more sulphate group C2-O-S in the 3,6-anhydrogalactose unit, circled in solid line in Fig. 11.

Water contact angle measurements is a simple, rapid, and inexpensive method which gives an indication of a surface's hydrophobicity. The measurement is, however, limited, which can lead to discrepancies in the results due to wetness of the sample surface and surface morphology, e.g., roughness.

Water contact angle is the measure of wettability. When a drop of water is placed on a surface, the liquid will interact differently depending on the intramolecular bonds in the material of the surface. There will be an angle formed between the liquid and the solid surface, and this angle is called the contact angle. The contact angle can be divided into static, dynamic, and roughness corrected contact angles.

The water contact angle may be measured by an optical tensiometer. It comprises a camera, a dispenser, sample stage and a light source.

The most common method of measurement of water contact angle is the sessile drop method. A drop of distilled water is placed on the sample surface. A direct measurement is taken by the camera and the angle to the surface is assessed. If the angle of the droplet is below 90°, then the surface is said to be wetted. This means that the water drop will be spread out on the surface. Conversely if the water contact angle is more than 90°, the surface is not wetted. The water drop will stay on the surface as a bead and the surface is said to be showing hydrophobic properties. For the experiment discussed in the following, a cut-off value 65° was chosen for the definition of hydrophobicity.

### Examples and experiments

The two types of carrageenan that were investigated are kappa- and iota-carrageenan. To achieve the desired hydrophobic surface, modification of carrageenan with different types of azetidinium salts has been evaluated. Azetidinium salts are suitable for this modification due to their selective reaction with sulphate groups and depending on which salt that is used, the hydrophobicity can be increased.

Previous studies have demonstrated that the modification of crystalline nanocellulose with azetidinium salts effectively enhances its water retention properties. Moreover, the versatility of these salts lies in their capability to alter the R-groups within their structure. Therefore, considering these factors, azetidinium salts present a favourable option for the desired hydrophobic enhancing modification. To enhance the mechanical properties of carrageenan films, plasticizers may be used. It has been found that glycerol and sorbitol are particularly suitable for use as plasticizers in the present context.

For the experiments, iota- and kappa-carrageenan powders of commercial grade were purchased from Sigma-Aldrich and used without further purification.

The azetidinium salts, M-Az, DHA-Az and DAL-Az were prepared in the way proposed in: P. Rosendahl "Design and synthesis of azetidinium salts for chemical modification of nanocrystalline cellulose", 2017, available from: https://www.semanticscholar.org/paper/Design-and-synthesis-of-azetidinium-salts-for-of-Rosendahl/590db9db8230040927669310ede075f84d2295ee . In brief, the preparation was made by reacting epichlorohydrin with three different secondary amines. Alternative ways of preparing the azetidinium salts, which may also be used, are known e.g. from "Isotropic Gels of Cellulose Nanocrystals Grafted with Dialkyl Groups: Influence of Surface Group Topology from Nonlinear Oscillatory Shear" by Wojno, S., Sonker, A. K., Feldhusen, J., Westman, G. and Kádár R.

The plasticizers, sorbitol and glycerol were purchased from Sigma-Aldrich.

Iota- and kappa-carrageenan were dissolved in deionized water (1 wt%) in a vial. The vial was then sealed with a lid and transferred to a heating block at 70°C for two hours. In absence of magnetic stirrer's, the vials were instead stirred with a glass rod after one hour. After the total time of two hours the sample solutions were transferred to plastic petri dishes. The plastic petri dishes were then left in contact with surrounding air to let the water in the films evaporate for a minimum of 48 hours.

The different samples prepared are listed in Table 2. Films were prepared both with and without azetidinium salt and plasticizers according to the methods described below.

**Table 2: Overview of all samples.**

| Sample name | Type of carrageenan | Azetidinium salt (molar ratio) | Plasticizer (wt%) |
|---|---|---|---|
| 1 | Kappa | - | - |
| 2 | Kappa | M-Az (1:1) | - |
| 3 | Kappa | M-Az (2:1) | - |
| 4 | Kappa | DHA-Az (1:1) | - |
| 5 | Kappa | DHA-Az (2:1) | - |
| 6 | Kappa | DAL-Az (1:1) | - |
| 7 | Kappa | DAL-Az (2:1) | - |
| 8 | Kappa | - | Glycerol (45) |
| 9 | Kappa | - | Glycerol (20) |
| 10 | Kappa | - | Sorbitol (45) |
| 11 | Kappa | - | Sorbitol (20) |
| 12 | Iota | - | - |
| 13 | Iota | M-Az (1:1) | - |
| 14 | Iota | M-Az (2:1) | - |
| 15 | Iota | DHA-A_{Z} (1:1) | - |
| 16 | Iota | DHA-A_{Z} (2:1) | - |
| 17 | Iota | DAL-Az (1:1) | - |
| 18 | Iota | DAL-Az (2:1) | - |
| 19 | Iota | - | Glycerol (45) |
| 20 | Iota | - | Glycerol (20) |
| 21 | Iota | - | Sorbitol (45) |
| 22 | Iota | - | Sorbitol (20) |
| 23 | Iota | M-Az (1:1) | Glycerol (45) |
| 24 | Iota | M-Az (1:1) | Glycerol (20) |
| 25 | Iota | M-Az (1:1) | Sorbitol (45) |
| 26 | Iota | M-Az (1:1) | Sorbitol (20) |
| 27 | Iota | DHA-Az (1:1) | Glycerol (45) |
| 28 | Iota | DHA-Az (1:1) | Glycerol (20) |
| 29 | Iota | DHA-Az (1:1) | Sorbitol (45) |
| 30 | Iota | DHA-Az (1:1) | Sorbitol (20) |
| 31 | Iota | DAL-Az (1:1) | Glycerol (45) |
| 32 | Iota | DAL-Az (1:1) | Glycerol (20) |
| 33 | Iota | DAL-Az (1:1) | Sorbitol (45) |
| 34 | Iota | DAL-Az (1:1) | Sorbitol (20) |

Addition of azetidinium salt was made to selected carrageenan solutions. All three salts were weighed with two different ratios in respect to the sulphate content in the two kappa-and iota-carrageenan used. Two molar ratios between sulphate and salt, 1: 1 and 2:1, respectively, were used. The different compositions of the samples are shown in Table 2.

Further, two different concentrations of glycerol and sorbitol were used as plasticizers. The used concentrations were 20 wt% and 45 wt% (based on the dry weight of the carrageenan powder). Plasticizers of the selected concentrations (20 or 45 wt%) were added to the prepared kappa-, and iota-carrageenan solutions and films were prepared according to the method described above.

### Characterisation

To analyze the samples two techniques were employed: Fourier Transform Infrared Spectroscopy (FTIR) and water contact angle measurements. FTIR was used to detect the possibility of chemical reaction and water contact angle was used to approximate the hydrophobicity of the formed films.

FTIR was recorded on two different PerkinElmer spectroscometers, namely PerkinElmer Frontier Fourier Transform Infrared Spectrometer and PerkinElmer Precisely Spectrum 100 FTIR Spectrometer. Experiments of FTIR were performed at room temperature and recorded at 4000-400 cm⁻¹ with 16 scans. All spectra were normalized to the peak of maximum absorbance (in the peak range 1024-1035cm⁻¹).

To measure the water contact angle a Krüss DSA 100 was used at room temperature. The measurements of water contact angle were taken at 0, 30 respectively 60 seconds to see how the hydrophobicity of the films performed over time. The cut of value of 65° was used to evaluate if the films were hydrophobic. Some values in the measurements show an increase in angle after time, which can be due to the machine's sensitivity or unevenness on the film surface.

To see if the films had obtained a good water barrier for six minutes, a drop of deionized water was placed on a selection of films. After six minutes the films were evaluated to see whether the water drop remained on the film surface or if the drop had passed through the film.

### Results and discussion

In Figs. 11 and 12 results from FTIR measurements on films from iota-carrageenan functionalised with different azetidinium salts at different molar ratios are presented. In both figures it can be seen a change in frequency around the peak for C2-O-S in anhydrogalactose unit for all films functionlised with azetidinium salts in comparison to the reference film, i.e. iota-carrageenan film without any azetidinium salt. This could be an indication that a reaction has occurred between carrageenan and the azetidinium salts. Subsequently, a change in frequency implies the formation of a new bond and further underlines that a reaction has occurred. It can also be deduced from Figs. 11 and 12 that the change in frequency is larger in Fig. 11, showing the films functionalized at a 1:1 molar ratio. This can be interpreted as a higher molar ratio being more favorable than a lower molar ratio. Additionally, it may be noted that no change in frequency corresponding to the C4-O-S in galactose region can be observed in Fig. 11 and Fig. 12.

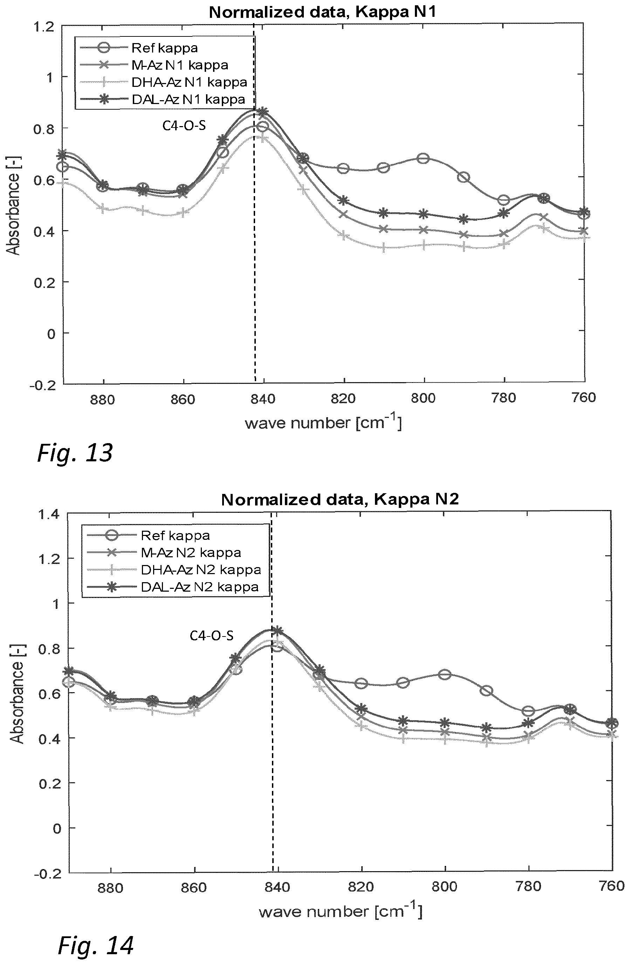

Figs. 13 and 14 show the FTIR results for the kappa-carrageenan samples. In kappa-carrageenan the C2-O-S bond is absent and consequently there should not be a peak at this frequency for any of the samples. Nevertheless, a small peak can be seen at this frequency in the reference sample. The reason for this peak could be due to impurities in the sample. From Figs. 13 and 14 it can be seen that there is no change in frequency in the C4-O-S region. This implies that there is no significant reaction between kappa-carrageenan and azetidinium salt.

Further, water contact angles for the samples were measured and evaluated for the kappa- and iota-carrageenan films with added salts.

In Table 3 results from the samples of kappa-carrageenan functionalized with azetidinium salt are presented. Overall, the films based on iota-carrageenan (see Table 4 below) seem to exhibit a higher hydrophobicity than the kappa-carrageenan films.

**Table 3: Results from water contact angle measurements on kappa-carrageenan films functionalized with azetidinium salt. Percentual change compared to initial value in parentheses.**

| Sample Kappa | CA [°] (0s) | CA [°] (30s) | CA [°] (60s) |
|---|---|---|---|
| Reference | 81,93 | 40,37 (-51%) | 42,38 (-48%) |
| M-Az (1:1) | 67,42 | 43,60 (-35%) | 38,62 (-43%) |
| M-Az (2:1) | 58,01 | 39,92 (-31%) | 36,12 (-38%) |
| DHA-Az (1:1) | 60,00 | 56,66 (-6%) | 52,94 (-12%) |
| DHA-Az (2:1) | 60,46 | 52,59 (-13%) | 51,25 (-15%) |
| DAL-Az (1:1) | 59,78 | 47,40 (-21%) | 63,23 (+6%) |
| DAL-Az (2:1) | 84,52 | 62,86 (-26%) | 64,98 (-23%) |

From these measurements, the following may be noted:
- None of the kappa-carrageenan films have a hydrophobicity which reach the cut-off value of 65° after 60s.
- Nevertheless, most of the samples have a greatly improved hydrophobicity after 60 seconds compared to the reference sample, i.e. the sample without any added azetidinium salt. Specifically, this applies to all the samples except the ones containing M-Az.
- Further, most of the samples have a greatly improved hydrophobicity also after 30 seconds compared to the reference sample, i.e. the sample without any added azetidinium salt. Specifically, this applies to all the samples except the one containing M-Az with a molar ratio of 2:1.

- The samples with DAL-Az had the highest hydrophobicity.
- All the samples have a much more stable development over time than the reference sample. In the reference sample, the angle after 30 and 60 seconds is less than 50% of the original reading (at 0s). For all the other samples, containing azetidinium salt, the decrease in angle is less than 1/3 at both 30 and 60 seconds.

Thus, it may be concluded that the use of azetidinium salt in the kappa-carrageenan film in most cases improves the hydrophobicity of the film, and at least makes the hydrophobicity/contact angle more stable over time, indicating an improved resistance to water, and consequently improved barrier and protection properties of the film.

In table 4, the measurement results for sample films of iota-carrageenan with different azetidinium salts are shown.

**Table 4: Results from water contact angle measurements on iota-carrageenan films functionalized with azetidinium salt. Percentual change compared to initial value in parentheses.**

| Sample Iota | CA [°] (0s) | CA [°] (30s) | CA [°] (60s) |
|---|---|---|---|
| Reference | 89,91 | 65,17 (-28%) | 55,38 (-38%) |
| M-Az (1:1) | 98,72 | 88,19 (-11%) | 83,20 (-16%) |
| M-Az (2:1) | 89,91 | 82,99 (-8%) | 87,12 (-3%) |
| DHA-Az (1:1) | 99.46 | 98,62 (-1%) | 93,32 (-6%) |
| DHA-Az (2:1) | 71,22 | 66,58 (-7%) | 64,04 (-10%) |
| DAL-Az (1:1) | 95,74 | 74,33 (-22%) | 77,24 (-19%) |
| DAL-Az (2:1) | 98,09 | 76,16 (-22%) | 66,79 (-32%) |

From these measurements, the following may be noted:
- Essentially all the samples containing azetidinium salt reaches the cut-off value of 65° after 60s, whereas the value for the reference sample (not containing any azetidinium salt) is much below this value.
- All of the azetidinium salts increase the hydrophobicity compared to the reference sample.
- All films exhibit a rather stable hydrophobicity during the studied time interval, and the percentual change in contact angle over time is much lower for all the tested samples than for the reference sample.
- M-Az and DHA-Az seem to have the most stable hydrophobicity.
- Additionally, the DHA-Az film seems to exhibit the highest contact angle in the molar ratio 1:1.
- The other two salts M-Az and DAL-Az show that they are over the cut-off value and formed transparent films.

Thus, it may be concluded that the use of azetidinium salt in the iota-carrageenan film improves the hydrophobicity of the film, and also makes the hydrophobicity/contact angle more stable over time, indicating an improved resistance to water, and consequently improved barrier and protection properties of the film.

In table 5, the measurement results for sample films of kappa- and iota-carrageenan films with added plasticizer, and without any azetidinium, are shown.

**Table 5: Results of water contact angle measurements of carrageenan films with added plasticizers. Kappa- and iota references are the films without any addition of plasticizer. Percentual change compared to initial value in parentheses.**

| Sample | CA [°] (0sec) | CA [°] (30sec) | CA [°] (60sec) |
|---|---|---|---|
| Kappa reference | 81.93 | 40.37 (-51%) | 42.38 (-48%) |
| Kappa Glycerol 45% | 48.37 | 39.62 (-18%) | 39.34 (-19%) |
| Kappa Glycerol 20% | 106.99 | 82.20 (-23%) | 72.43 (-32%) |
| Kappa Sorbitol 45% | 68.50 | 42.61 (-38%) | 37.30 (-46%) |
| Kappa Sorbitol 20% | 72.58 | 58.33 (-20%) | 44.12 (-39%) |
| Iota reference | 89.91 | 65.17 (-28%) | 55.38 (-38%) |
| Iota Glycerol 45% | 91.68 | 76.00 (-17%) | 73.16 (-20%) |
| Iota Glycerol 20% | 93.73 | 88.23 (-6%) | 83.10 (-11%) |
| Iota Sorbitol 45% | 87.96 | 84.44 (-4%) | 83.61 (-5%) |
| Iota Sorbitol 20% | 108.33 | 89.48 (-17%) | 82.18 (-24%) |

The contact angle results from the addition of plasticizers are shown in Table 5. For the kappa-carrageenan films only one film met the contact angle cut-off value namely the film with a concentration of 20 wt% glycerol. It may further be noted that some of the kappa-carrageenan films with plasticizer have a greatly improved hydrophobicity compared to the reference (without any plasticizer), whereas the other have essentially the same hydrophobicity. Still further, all the samples with plasticizer exhibit a rather stable hydrophobicity during the studied time interval, and the percentual change in contact angle over time is much lower for all the tested samples than for the reference sample.

Regarding the iota-carrageenan films, they all exhibit a surprisingly good hydrophobicity as they all fulfil the cut-off value of 65°. The decrease in water contact angle seems to be smallest for iota film with 45 wt% added sorbitol over the time span of 60s, while the iota film with 20 wt% sorbitol demonstrates to be more hydrophobic at the initial measurement but becomes rapidly less hydrophobic after 60s.

For the iota-carrageenan films, all the samples with plasticizers met the contact angle cut-off value, whereas the reference sample did not reach this criterion. It may further be noted that all of the iota-carrageenan films with plasticizer have a greatly improved hydrophobicity compared to the reference (without any plasticizer). Still further, all the samples with plasticizer exhibit a rather stable hydrophobicity during the studied time interval, and the percentual change in contact angle over time is much lower for all the tested samples than for the reference sample.

Thus, it may be concluded that the use of a plasticizer in the kappa- and iota-carrageenan film improves the hydrophobicity of the film, and in particular this is the case for iota-carrageenan films, and also makes the hydrophobicity/contact angle more stable over time, indicating an improved resistance to water, and consequently improved barrier and protection properties of the film.

From the results, both glycerol and sorbitol seem to increase the hydrophobicity when comparing to the carrageenan films without any added plasticizer. This can be due to the formation of double helices which are formed during the film formation. These double helices can then align with each other and this could possibly lead to stronger hydrogen bonds to the plasticizer than to the water resulting in the films becoming more water repellent (19).

In table 6, the measurement results for sample films of iota-carrageenan films with both added plasticizer and added M-Az are shown. As a first reference, the previously measured iota-carrageenan sample without both plasticizer and azetidinium is presented, and as a second reference, iota-carrageenan with M-Az but without plasticizer is used.

**Table 6: Results of water contact angle from iota carrageenan films functionalized with M-Az and added plasticizers. Percentual change compared to initial value in parentheses.**

| Sample | CA [°] (0sec) | CA [°] (30sec) | CA [°] (60sec) |
|---|---|---|---|
| Reference | 89,91 | 65,17 (-28%) | 55,38 (-38%) |
| M-Az iota reference | 98.72 | 88.19 (-11%) | 83.20 (-16%) |
| M-Az Glycerol 45% | 81.80 | 72.27 (-12%) | 74.87 (-8%) |
| M-Az Glycerol 20% | 81.34 | 43.96 (-46%) | 40.89 (-50%) |
| M-Az Sorbitol 45% | 46.18 | 34.69 (-25%) | 33.51 (-27%) |
| M-Az Sorbitol 20% | 95.94 | 90.33 (-6%) | 76.21 (-21%) |

Table 6 presents the results of iota-carrageenan films with the addition of M-Az and the plasticizer. The only films that meet the contact angle cut-off requirement are the ones having an addition of 45 wt% glycerol or 20 wt% sorbitol. This is interesting since the glycerol in this case seems to work best at a higher concentration and sorbitol at the lower concentration. If we compare these to the M-Az iota-reference film the hydrophobicity is lower in all cases where plasticizer has been added. However, compared to the reference without plasticizer and salt, the samples with 45 wt% glycerol and 20 wt% sorbitol have a much greater hydrophobicity.

It may be concluded that for many cases it is possible to use a combination of plasticizer and azetidinium salt, and in particular M-Az, and still obtain a great improvement in hydrophobicity, in addition to other improvements, such as in term of the mechanical properties.

In table 7, the measurement results for sample films of iota-carrageenan films with both added plasticizer and added DHA-Az are shown. As a first reference, the previously measured iota-carrageenan sample without both plasticizer and DHA-Az is presented, and as a second reference, iota-carrageenan with DHA-Az but without plasticizer is used.

**Table 7: Results of water contact angle from iota carrageenan films functionalized with DHA-Az and added plasticizers. Percentual change compared to initial value in parentheses.**

| Sample | CA [°] (0sec) | CA [°] (30sec) | CA [°] (60sec) |
|---|---|---|---|
| Reference | 89,91 | 65,17 (-28%) | 55,38 (-38%) |
| DHA-Az iota reference | 99.46 | 98.62 (-1%) | 93.32 (-6%) |
| DHA-Az Glycerol 45% | 74.42 | 66.27 (-11%) | 55.20 (-26%) |
| DHA-Az Glycerol 20% | 76.73 | 70.22 (-8%) | 66.37 (-14%) |
| DHA-Az Sorbitol 45% | 72.05 | 70.09 (-3%) | 69.08 (-4%) |
| DHA-Az Sorbitol 20% | 51.35 | 47.43(-8%) | 45.55 (-11%) |

Regarding the result of the films functionalized with DHA-Az and added plasticizer (Table 7) the hydrophobicity seems to have decreased generally when compared to the DHA-Az iota-reference film. Addition of 20wt% glycerol or 45wt% sorbitol are the only samples that meet the requirement. This is interesting since the glycerol in this case seems to work best at a lower concentration and sorbitol at the higher concentration. This is also the opposite effect than what is seen for M-Az. If we compare the measured samples to the DHA-Az iota-reference film, the hydrophobicity is lower in all cases where plasticizer has been added. However, compared to the reference without plasticizer and salt, the samples with 20 wt% glycerol and 45 wt% sorbitol have a much greater hydrophobicity.

It may be concluded that for many cases it is possible to use a combination of plasticizer and azetidinium salt, and in particular DHA-Az, and still obtain a great improvement in hydrophobicity, in addition to other improvements, such as in term of the mechanical properties.

In table 8, the measurement results for sample films of iota-carrageenan films with both added plasticizer and added DAL-Az are shown. As a first reference, the previously measured iota-carrageenan sample without both plasticizer and DAL-Az is presented, and as a second reference, iota-carrageenan with DAL-Az but without plasticizer is used.

**Table 8: Results of water contact angle from iota films carrageenan functionalized with DAL-Az and added plasticizers. Percentual change compared to initial value in parentheses.**

| Sample | CA [°] (0sec) | CA [°] (30sec) | CA [°] (60sec) |
|---|---|---|---|
| Reference | 89,91 | 65,17 (-28%) | 55,38 (-38%) |
| DAL-Az iota reference | 95.74 | 74.33 (-22%) | 77.24 (-19%) |
| DAL-Az Glycerol 45% | 111.10 | 82.96 (-25%) | 76.03 (-32%) |
| DAL-Az Glycerol 20% | 80.71 | 88.22 (+9%) | 71.31 (-12%) |
| DAL-Az Sorbitol 45% | 79.62 | 62.03 (-22%) | 56.98 (-28%) |
| DAL-Az Sorbitol 20% | 89.42 | 80.37 (-10%) | 74.29 (-17%) |

The result of the films functionalized with DAL-Az and added plasticizer are presented in Table 8. The sample with an addition of 45wt% glycerol has the highest hydrophobicity, but it also decreases the fastest over the time span of 60s. Addition of sorbitol 45% was the only film that did not meet the requirement of 65°.

If we compare the measured samples to the DAL-Az iota-reference film the hydrophobicity is lower in all cases where plasticizer has been added. However, compared to the reference without plasticizer and salt, all the samples with plasticizer and DAL-Az have a much greater hydrophobicity and also an increased stability for the hydrophobicity.

It may be concluded that for many cases it is possible to use a combination of plasticizer and azetidinium salt, and in particular DAL-Az, and still obtain a great improvement in hydrophobicity, in addition to other improvements, such as in term of the mechanical properties.

In further experimental tests, an ocular and tactile inspection of the film samples were made.

All samples with azetidinium salts could be formed as films. However, some of the samples were brittle, uneven, and/or difficult to remove from the plastic petri dishes. The ocular and tactile film properties were essentially the same for the reference samples containing only carrageenan and water. However, the iota-carrageenan films modified with M-Az and DAL-Az seemed to have the best mechanical film properties as these were not as brittle.

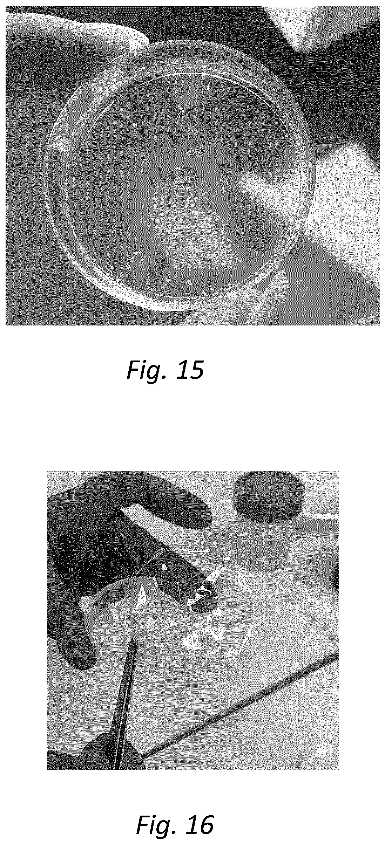

The addition of plasticizers to the carrageenan films without azetidinium salt seemed to improve the ocular and tactile film properties in terms of durability and elasticity (see also Fig. 16). The majority of the films were easier to remove from the plastic petri dish and intact films were formed. The kappa-carrageenan films seemed to have slightly better mechanical properties than iota-carrageenan films with the plasticizers.

Although the addition of plasticizer to iota-carrageenan films seemed to improve the mechanical properties, the addition of azetidinium salts to these films did not seem to have the same apparent effect.

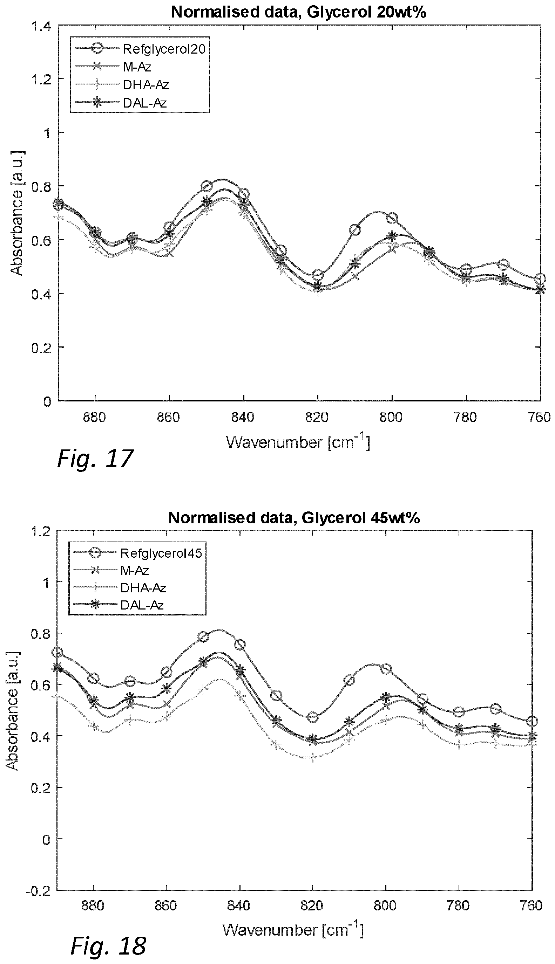

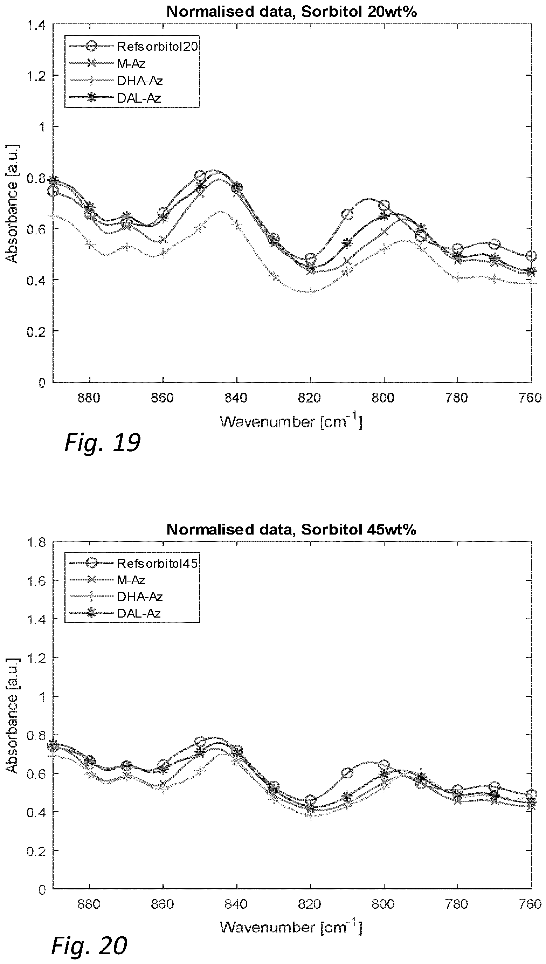

Furthermore, to ensure that the plasticizers did not interfere with the reaction of iota-carrageenan and azetidinium salt, FTIR was performed on these films. The result of these measurements are shown in Figs. 17-20. The FTIR spectra show the same indication of reaction. Hence, no conflict between the azetidinium salts and the added plasticizers were observed.

For certain products, as in the above-discussed catheter product, the package will be exposed to water or other wetting liquids only for a very limited time. This time could comprise the time needed for wetting and activation of the hydrophilic medical device, which would typically take only a few seconds, or at least less than a minute, and possibly also the time during which catheterization takes place, which would normally be at most 5 minutes for intermittent catheterization. Thereafter, the package and catheter may normally be discarded. Thus, in additional experimental tests, the ability of the package to resist water for 6 minutes were tested. Since this type of catheter product is intended to be used during six minutes after activation, it may be of some importance that the films can provide a water barrier for this time interval.

Based on previous results on the water contact angle measurements and the ocular inspection it was decided to evaluate all kappa- and iota-carrageenan films with added plasticizer as well as the iota-carrageenan films functionalized with azetidinium salt and added plasticizer. Except from the evaluation was the iota-carrageenan films functionalized with DHA-Az and with added sorbitol as that composition did not form good enough films to be evaluated in this assessment.

In Table 9 the result of the kappa- and iota-carrageenan films with added plasticizer is shown. The films that provided a water barrier for six minutes is marked with "YES", whereas films that did not meet the water barrier requirement are marked with "NO". In Table 10 the results of the functional water-resistant evaluation of the iota-carrageenan films functionalized with azetidinium salt and added plasticizer are shown in the same way.

**Table 9: Results for the final water barrier evaluation for kappa- and iota-carrageenan films with added plasticizer. The films that could uphold a water barrier for six minutes is marked with "YES", and the films that could not is marked with "NO ".**

| Sample | Upholds water barrier |
|---|---|
| Kappa Glycerol 45% | NO |
| Kappa Glycerol 20% | YES |
| Kappa Sorbitol 45% | YES |
| Kappa Sorbitol 20% | NO |
| Iota Glycerol 45% | NO |
| Iota Glycerol 20% | YES |
| Iota Sorbitol 45% | NO |
| Iota Sorbitol 20% | NO |

**Table 10: Results for the final water barrier evaluation of iota-carrageenan films functionalized with azetidinium salt and added plasticizer. The films that could uphold a water barrier for six minutes is marked with "YES" and the films that could not is marked with "NO".**

| Sample | Upholds water barrier |
|---|---|
| M-Az Glycerol 45% | YES |
| M-Az Glycerol 20% | YES |
| M-Az Sorbitol 45% | YES |
| M-Az Sorbitol 20% | NO |
| DHA-Az Glycerol 45% | YES |
| DHA-Az Glycerol 20% | NO |
| DAL-Az Glycerol 45% | YES |
| DAL-Az Glycerol 20% | YES |
| DAL-Az Sorbitol 45% | NO |
| DAL-Az Sorbitol 20% | NO |

It may be noted that some of the films that did not manage to reach the cut-off value of 65° in the water contact angle measurement could still uphold a water barrier for six minutes. One possible reason for this could be due to the water contact angle measurement being very sensitive and the surface of film could be uneven resulting in different results depending on where the drop is placed. Additionally, a possible wetness of the sample surface during the contact angle measurement could also be an explanation for this deviation.

It has thus been shown that carrageenan can be used to form a film, useful in packages as a protective layer. In particular, the film could be used as a barrier or protection against water and other liquids. These properties are also greatly improved when carrageenan is used in combination with a plasticizer and/or a salt, and in particular an azetidinium salt, and most particularly DHA-Az.

To improve hydrophobicity, the approach has been to functionalize carrageenan with salt, and in particular the three different azetidinium salts discussed above. FTIR-spectroscopy of the carrageenan films functionalized with different azetidinium salts indicated that the salts react with iota-carrageenan but not with kappa-carrageenan. Modified iota-carrageenan has a particularly high hydrophobicity. Out of the three azetidinium salts, the iota-film modified with DHA-Az had the best hydrophobic properties. However, the other two tested salts form even films and also show good hydrophobic properties.

Plasticizers can be added to improve the mechanical properties of the films. The plasticizers were found to work best with the kappa-carrageenan films, but an overall improvement of the films for both carrageenan types could be observed.

It was also noted that the hydrophobicity of the films was improved with the addition of plasticizers compared to the films without plasticizer.

The compatibility of plasticizers and iota-carrageenan modified with azetidinium salt were also studied. This showed a small indication of improved mechanical properties. The water contact angle measurements seem to decrease compared to films functionalized with azetidinium salt without plasticizer. However, several films still have a very good hydrophobicity.

The six minutes functional water-resistant evaluation that was performed, further proved a good capability of serving as a protection and barrier for many of the samples. Out of the iota- and kappa-carrageenan films with added plasticizer only three films upheld a sufficient water barrier for six minutes. The iota-carrageenan films functionalized with azetidinium salt and added plasticizer results show deviation from the water contact angle results. The majority of these films were able to maintain a water barrier for six minutes.

The person skilled in the art realizes that the present invention by no means is limited to the preferred embodiments described above. On the contrary, many modifications and variations are possible within the scope of the appended claims. For example, the film could be arranged on other types of substrates than paper, such as other cellulose based materials, bioplastic materials, etc. The package could also be used for other types of urinary catheter products, or for catheter intended for other purposes, and also for other types of hydrophilic and non-hydrophilic medical devices. Such and other obvious modifications must be considered to be within the scope of the present invention, as it is defined by the appended claims. It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting to the claim. The word "comprising" does not exclude the presence of other elements or steps than those listed in the claim. The word "a" or "an" preceding an element does not exclude the presence of a plurality of such elements. Further, a single unit may perform the functions of several means recited in the claims.

## Claims

1. A medical device assembly comprising a medical device, and preferably a hydrophilic medical device, and a package forming an interior cavity, the cavity arranged to enclose at least a part of said medical device, wherein the package comprises an substrate made of a bio-based material and a film made of carrageenan arranged on said substrate.

2. A medical device assembly comprising a hydrophilic urinary catheter and a package forming an interior cavity, the cavity arranged to enclose at least a part of said hydrophilic medical device, wherein the package comprises an outer substrate made of a bio-based material, such as paper, and a hydrophobic film made of linear sulfated polysaccharides, such as carrageenan, wherein the films is arranged facing the interior cavity.

3. The medical device assembly of claim 1 or 2, wherein the bio-based material is a cellulose based material, and preferably based on paper or seaweed.

4. The medical device assembly of any one of the preceding claims, wherein the carrageenan is at least one of kappa-carrageenan and iota-carrageenan, and preferably iota-carrageenan.

5. The medical device assembly of any one of the preceding claims, wherein film further comprises an azetidinium salt bonded to or mixed with the carrageenan.

6. The medical device assembly of claim 5, wherein the azetidinium salt and the carrageenan form a cross-linked network.

7. The medical device assembly of any one of the claims 5-6, wherein the azetidinium salt comprises at least one of morpholine-azetidinium salt (M-Az), 1,1-dihexyl-3-hydroxy-azetidinium salt (DHA-Az), and 1,1-diallyl-3-hydroxy-azetidinium salt (DAL-Az).

8. The medical device assembly of any one of the claims 5-7, wherein a molar ratio between sulphate in the carrageenan and the azetidinium salt is in the range 1:1 to 2:1.

9. The medical device assembly of any one of the preceding claims, wherein the film further comprises a plasticizer.

10. The medical device assembly of claim 9, wherein the plasticizer is at least one of a sugar alcohol and a triol, and preferably at least one of sorbitol and glycerol, and most preferably glycerol.

11. The medical device assembly of claim 9 or 10, wherein the film comprises 5-50 wt% of plasticizer, and preferably 15-50 wt%, and most preferably 20-45 wt%.

12. The medical device assembly of any one of the preceding claims, wherein the film is hydrophobic and forms a water barrier.

13. The medical device assembly of any one of the preceding claims, wherein the film is arranged as an inner film on the substrate, facing the interior cavity.

14. The medical device assembly of any one of the preceding claims, wherein the medical device is a hydrophilic catheter, and preferably a hydrophilic urinary catheter.

15. The medical device assembly of any one of the preceding claims, wherein the medical device further comprise a wetting fluid compartment arranged within said interior cavity.
